# EUROPEAN PATENT SPECIFICATION

(11) **EP 3 207 885 B1**
(45) Date of publication and mention of the grant of the patent: **22.05.2024**
(21) Application number: 17154422.4
(22) Date of filing: 02.02.2017
(51) Int. Cl.: A61B 17/29, A61B 90/70

(54) **MEDICAL INSTRUMENT WITH INSTRUMENT FLUSHING SYSTEM**
MEDIZINISCHES INSTRUMENT MIT INSTRUMENTENSPÜLSYSTEM
INSTRUMENT MÉDICAL AYANT UN SYSTÈME DE RINÇAGE

(30) Priority: 19.02.2016 US 201615048682
(43) Date of publication of application: 23.08.2017
(73) Proprietor: Arthrex, Inc, Naples, FL 34108-1945 (US)
(72) Inventor: WEBER, Robert McGregor, Chino Hills, FL 91709 (US)
(74) Representative: Westphal, Mussgnug & Partner Patentanwälte mbB

(56) References cited:
- EP-A1- 1 199 041
- US-A- 6 068 641
- US-A1- 2015 297 290
- US-B1- 6 419 688

## Description

### FIELD OF THE DISCLOSURE

The present disclosure generally relates to medical instruments, and more particularly to medical instruments with flushing systems for cleaning the instruments after use.

### BACKGROUND

Medical instruments used for surgical procedures are commonly flushed after use to remove any bodily tissue that may have accumulated within the instrument during use. The medical instruments may be disassembled to clean and remove the bodily tissue. Some medical instruments include flush systems to flush internal aspects of the medical instruments. However, many of these systems do not flush all tissue an blood from interior aspects of the devices. Thus, there remains a need for an efficient system for flushing internal aspects of medical instruments. Medical instruments with an outer housing, in which fluid flows from a proximal to a distal end of said elongated outer housing are known for example from US 6,419,688 B1, US 2015/0297290 A1, US 6 068 641 A and EP 1 199 041 A1.

### SUMMARY

The invention is defined by claims 1 and 9. Further embodiments of the invention are defined by the dependent claims.

A medical instrument with an elongated outer housing and inner drive shaft and including an instrument flushing system that enables the medical instrument to be effectively cleaned after use is disclosed. The medical instrument may be configured such that the instrument flushing system receives a flushing fluid through a port at a proximal end of the instrument and directs the flushing fluid through a flushing fluid plenum created between an inner surface of the elongated outer housing and an outer surface of the inner drive shaft. According to the invention the flushing fluid plenum is maintained by a standoff formed from a collar with one or more axially extending grooves. The instrument flushing system may include a seal configured to seal portions of a drive system in communication with the inner drive shaft and extending radially outward from a main body of the medical instrument.

The medical instrument according to the invention is formed from an elongated outer housing with a hollow inner plenum, whereby a distal end of the elongated outer housing includes an outer shaft distal opening. The medical instrument also includes a main body extending proximally from a proximal end of the elongated outer housing. The medical instrument includes an inner drive shaft positioned within the elongated outer housing and extending into a hollow cavity within the main body, whereby the inner drive shaft is sized such that a flushing fluid plenum exists in the hollow inner plenum of the elongated outer housing between an outer surface of the inner drive shaft and an inner surface of the elongated outer housing. The medical instrument also includes a drive system in communication with the inner drive shaft and extending radially outward from the main body, whereby the drive system is configured to move the inner drive shaft axially within the hollow inner plenum of the elongated outer housing to drive distally positioned working heads. The medical instrument also includes an instrument flushing system in communication with the hollow cavity within the main body and configured to pass flushing fluid through the hollow cavity within the main body and through the flushing fluid plenum in the hollow inner plenum of the elongated outer housing between the outer surface of the inner drive shaft and the inner surface of the elongated outer housing.

A method of cleaning a medical instrument according to the invention includes providing fluid to the medical instrument formed from an elongated outer housing with a hollow inner plenum, whereby a distal end of the elongated outer housing includes an outer shaft distal opening. The medical instrument includes a main body extending proximally from a proximal end of the elongated outer housing and an inner drive shaft positioned within the elongated outer housing and extending into a hollow cavity within the main body, whereby the inner drive shaft is sized such that a flushing fluid plenum exists in the hollow inner plenum of the elongated outer housing between an outer surface of the inner drive shaft and an inner surface of the elongated outer housing. The medical instrument includes a drive system in communication with the inner drive shaft and extending radially outward from the main body, whereby the drive system is configured to move the inner drive shaft axially within the hollow inner plenum of the elongated outer housing. The medical instrument includes an instrument flushing system in communication with the hollow cavity within the main body and configured to pass flushing fluid through the hollow cavity within the main body and through the flushing fluid plenum in the hollow inner plenum of the elongated outer housing between the outer surface of the inner drive shaft and the inner surface of the elongated outer housing. The method may also include enabling flush fluid to flow into the instrument flushing system.

An advantage of the medical instrument is that the instrument flushing system enables tissue particles and blood to be easily and effectively flushed from internal aspects of the medical instrument such as within the elongated outer housing.

Another advantage of the medical instrument is the standoff of the instrument flushing system within the elongated outer housing positions the inner drive shaft within the elongated outer housing while enabling flushing fluid to flow past the standoff by flowing through the axially extending grooves of the standoff.

Yet another advantage of the medical instrument is the seal surrounding the first drive handle protruding from the main body of the medical instrument eliminates or substantially eliminates leakage of the flushing fluid form the instrument flushing system.

Another advantage of the medical instrument is one or more ports in the instrument flushing system that may be configured to be releasably coupled to a flushing fluid supply source. The port may also be aligned axially with the hollow cavity in the main body and with the flushing fluid plenum in the elongated outer housing.

These and other embodiments are described in more detail below.

### BRIEF DESCRIPTION OF THE DRAWINGS

The accompanying drawings, which are incorporated in and form a part of the specification, illustrate embodiments of the presently disclosed invention embodiments of the disclosure and, together with the description, disclose the principles of the invention.
Figure 1 is a side view of a medical instrument including an instrument flushing system.
Figure 2 is a perspective view of the medical instrument of Figure 1.
Figure 3 is an exploded perspective view of the medical instrument of Figure 1.
Figure 4 is yet another exploded perspective view of the medical instrument of Figure 1 with a portion of the main body removed, exposing the hollow cavity within the main body.
Figure 5 is a perspective view of the inner drive shaft positioned within the elongated outer housing via a standoff having a plurality of ridges that extend outwardly in a radial direction according to the claimed invention.
Figure 6 is a perspective view of a portion of a seal used to seal a first drive handle extending from the main body.
Figure 7 is a flow diagram of a method of using the medical instrument including an instrument flushing system of Figure 1.
Figures 8 is a perspective view of the inner drive shaft positioned within the elongated outer housing via a standoff having a plurality of ridges that extend inwardly in a radial direction.
Figure 9 is an end view of the standoff shown in Figure 8.
Figure 10 is a perspective view of the inner drive shaft positioned within the elongated outer housing via a standoff having a plurality of ridges that extend inwardly in a radial direction and.
Figure 11 is an end view of the standoff shown in Figure 10.
Figure 12 is an end view of a standoff without a plurality of ridges.

### DETAILED DESCRIPTION

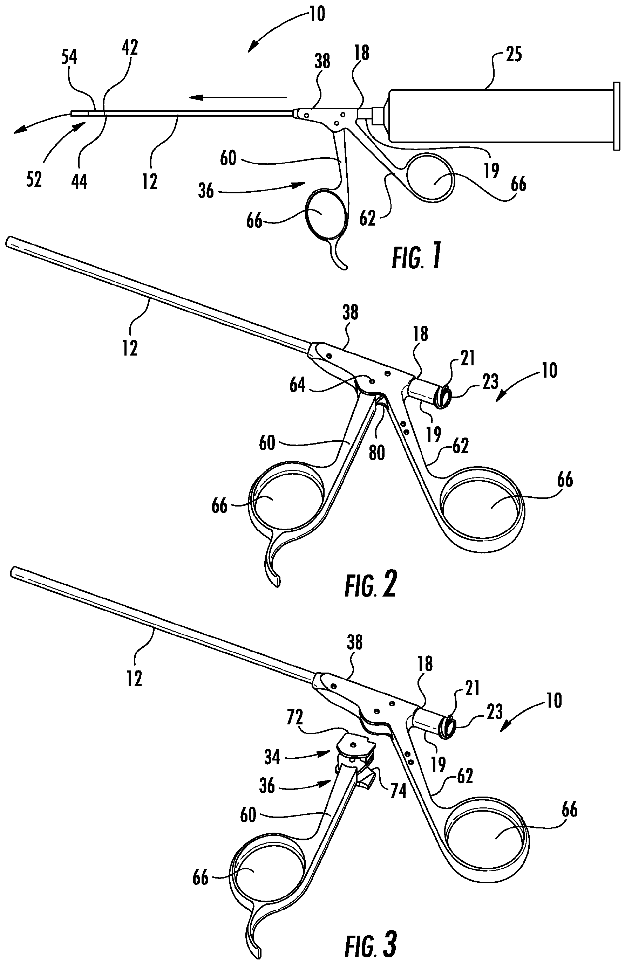

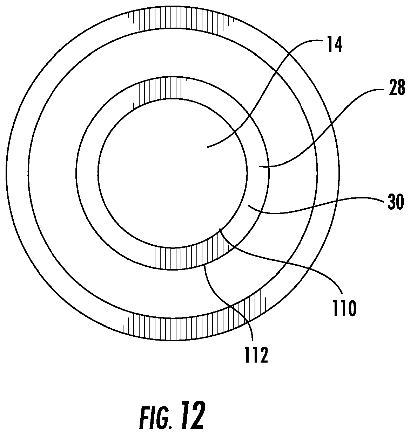

As shown in Figures 1-12, a medical instrument 10 with an elongated outer housing 12 and inner drive shaft 14 and including an instrument flushing system 16 that enables the medical instrument 10 to be effectively cleaned after use is disclosed. The medical instrument 10 may be configured such that the instrument flushing system 16 receives a flushing fluid through a port 18 at a proximal end 20 of the instrument 10 and directs the flushing fluid through a flushing fluid plenum 22 created between an inner surface 24 of the elongated outer housing 12 and an outer surface 26 of the inner drive shaft 14. The flushing fluid plenum 22 may be maintained by a standoff 28 formed from a collar 30 with one or more axially extending grooves 32. The instrument flushing system 16 may include a seal 34 to seal portions of a drive system 36 in communication with the inner drive shaft 14 and extending radially outward from a main body 38 of the medical instrument 10.

The medical instrument 10 may include an elongated outer housing 12 with a hollow inner plenum 40. A distal end 42 of the elongated outer housing 12 may include an outer shaft distal opening 44. A main body 38 may be coupled to a proximal end of the elongated outer housing 12. The main body 38 may extend proximally from a proximal end 46 of the elongated outer housing 12. The main body 38 may be formed from any appropriate material, such as, but not limited to, plastic and stainless steel. The main body 38 may include a hollow cavity 48 positioned therein and having any appropriate configuration. The proximal end 46 of the elongated outer housing 12 may extend into a distal end 50 of the hollow cavity 48.

An inner drive shaft 14 may be positioned within the elongated outer housing 12 and may extend into the hollow cavity 48 within the main body 38. The inner drive shaft 14 may be sized such that a flushing fluid plenum 22 exists in the hollow inner plenum 40 of the elongated outer housing 12 between an outer surface 26 of the inner drive shaft 14 and an inner surface 24 of the elongated outer housing 12 when the inner drive shaft 14 is positioned within the outer housing 12. The inner drive shaft 14 may be formed form any appropriate material, such as, but not limited to, plastic and stainless steel. The inner drive shaft 14 may have a generally cylindrical cross-sectional shape or other appropriate shape. As shown in Figure 1, the inner drive shaft 14 may include a one or more working heads 52 attached to a distal end 54 of the inner drive shaft 14. The working head 52 may be, but is not limited to being, one or more set of jaws, convention working heads or yet to be developed working heads.

The medical instrument 10 may include a drive system 36, as shown in Figures 1-4, in communication with the inner drive shaft 14 and extending radially outward from the main body 38. The drive system 36 may be configured to move the inner drive shaft 14 axially within the hollow inner plenum 40 of the elongated outer housing 12. The drive system 36 may be formed from a first drive handle 60 and a second drive handle 62 configured such that the first drive handle 60 is movable relative to the second drive handle 62 to move the inner drive shaft 14 axially. The first drive handle 60 may be coupled to a pivot pin 64 such that the first drive handle 60 may pivot about the pivot pin 64. Moving the first drive handle 60 towards the second drive handle 62 may cause the inner drive shaft 14 to move axially toward the distal end 42 of the elongated outer housing 12, which in turn may drive the working head 52. The second drive handle 62 may be rigidly coupled to the main body 38. The first drive handle 60 may be moved towards the second drive handle 62 that may be positioned closer towards the proximal end 20 of the instrument 10 than the first drive handle 62. The first drive handle 60 or the second drive handle 62, or both, may include a finger receptacle 66 configured to receive one or more fingers of a user's hand. The finger receptacle 66 may have any appropriate size and configuration.

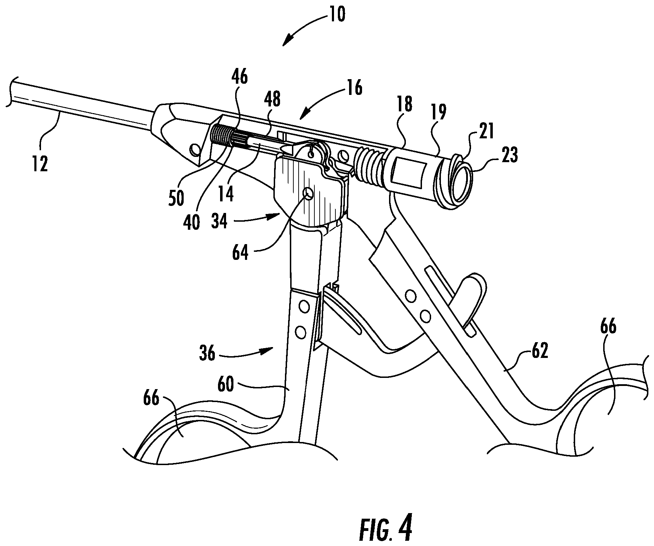

The medical instrument 10 may include an instrument flushing system 16, as shown in Figure 4, in communication with the hollow cavity 48 within the main body 38 and configured to pass flushing fluid through the hollow cavity 48 within the main body 38 and through the flushing fluid plenum 22 in the hollow inner plenum 40 of the elongated outer housing 12 between the outer surface 26 of the inner drive shaft 14 and the inner surface 24 of the elongated outer housing 12. The instrument flushing system 16 may be configured to supply flushing fluids through the interior aspects of the medical instrument 10 to remove bodily tissue from the medical instrument 10. The instrument flushing system 16 may include one or more ports 18 positioned at a proximal end 20 of the main body 38 of the medical instrument 10. The instrument flushing system 16 may be configured such that the port 18 is axially aligned with the hollow cavity 48 within the main body 38 and the flushing fluid plenum 22 between the elongated outer housing 12 and the inner drive shaft 14. As such, the instrument flushing system 16 is capable of successfully providing flushing action to inner aspects of the medical instrument 10 and to one or more working heads 52 attached to the distal end 54 of the inner drive shaft 14. The port 18 of the instrument flushing system 16 may be a releasable connection, as shown in Figure 4. The releasable connection may be, but is not limited to being, a LUER-LOK fitting 19. The releasable connection may be a female or male LUER-LOK fitting 19. The LUER-LOK fitting 19 may be releasably attached to the port 18, such as, but not limited to being, threadably attached. The LUER-LOK fitting 19 may include threads 21 on an end 23 of the LUER-LOK fitting 19.

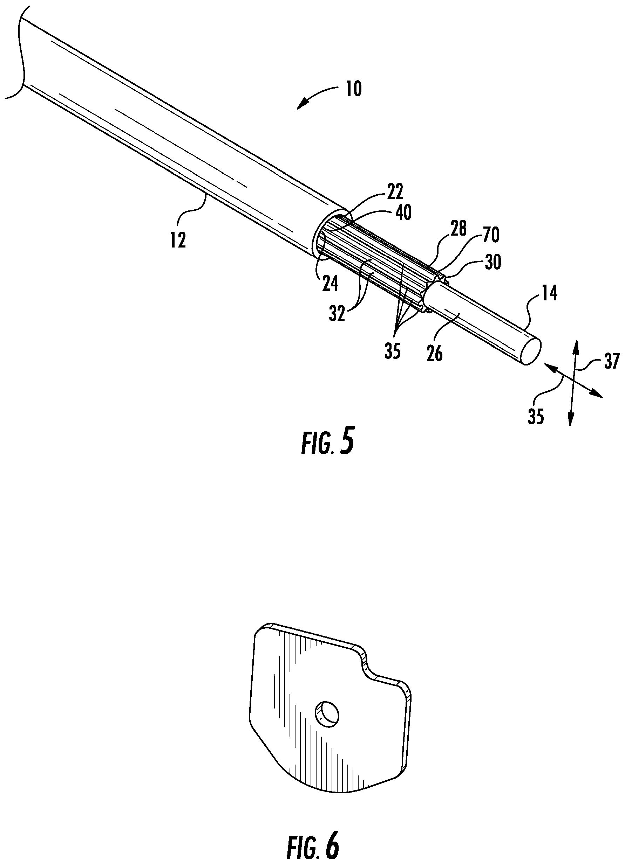

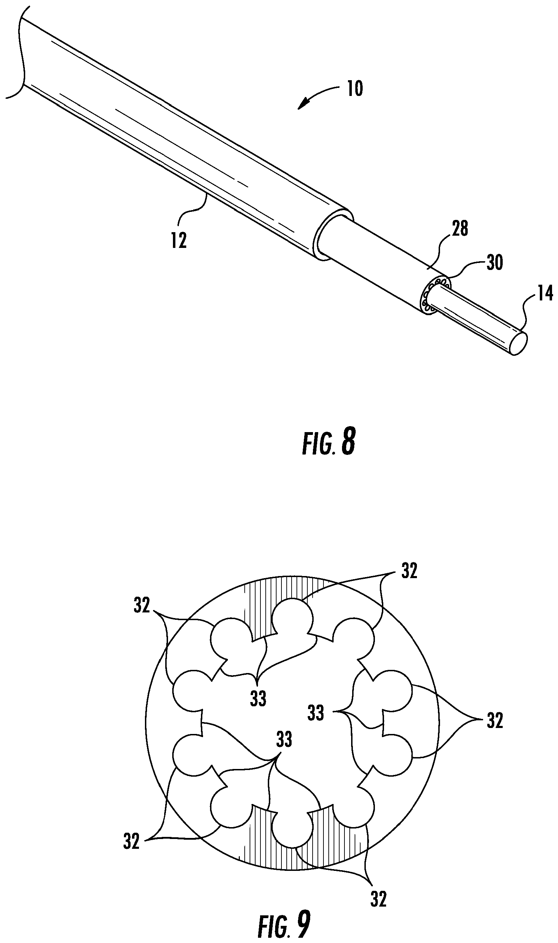

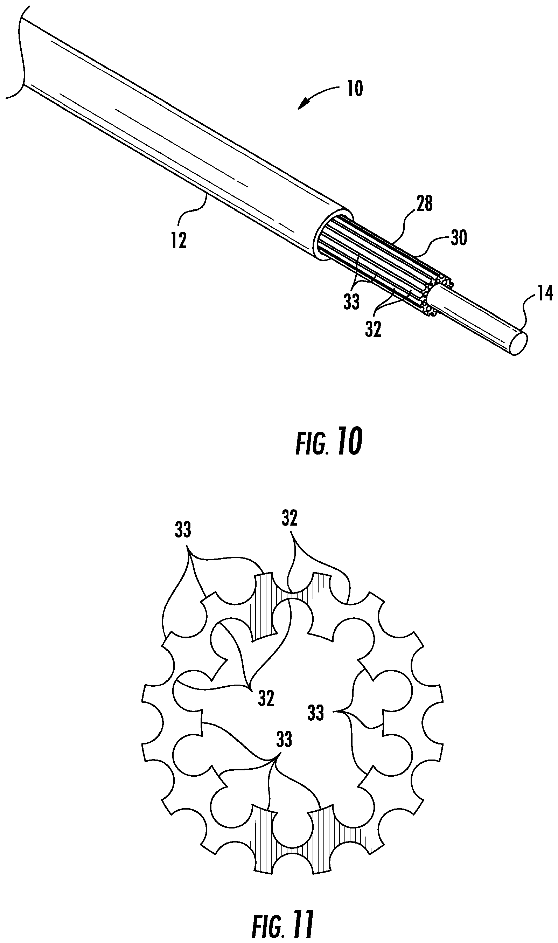

The instrument flushing system 16 includes one or more standoffs 28, as shown in Figures 4 and 5, positioned in the flushing fluid plenum 22. The standoffs 28 may be formed from different configurations, as shown, for example, in Figures 5, 8 and 9. Figure 5 discloses an embodiment according to the claimed invention, a configuration of the standoff 28 in which a plurality of axially extending grooves 32 are formed from a plurality of ridges 33 that extend outwardly in a radial direction 37. In an alternative embodiment of the disclosure, figures 8 and 9 disclose a configuration of the standoff 28 in which a plurality of axially extending grooves 32 are formed from a plurality of ridges 33 that extend inwardly in a radial direction 37. In a further alternative example of the disclosure, figures 10 and 11 disclose a configuration of the standoff 28 in which a plurality of axially extending grooves 32 are formed from a plurality of ridges 33 that extend outwardly in a radial direction 37 and a plurality of axially extending grooves 32 are formed from a plurality of ridges 33 that extend inwardly in a radial direction 37.

The instrument flushing system 16 according to an embodiment of the invention, as shown in Figure 5, includes one or more standoffs 28 extending between the outer surface 26 of the inner drive shaft 14 and the inner surface 24 of the elongated outer housing 12 to maintain the flushing fluid plenum 22. The standoff 28 need not contact both the outer surface 26 and the inner surface 24 at the same time. The standoff 28 of the instrument flushing system 16 is formed from a collar 30 extending radially outward from the outer surface 26 of the inner drive shaft 14 whereby the collar 30 includes one or more axially extending grooves 32 therein. The collar 30 may include a plurality of axially extending grooves 32. The axially extending grooves 32 may be formed from a plurality of ridges 33 that extend outwardly in a radial direction 37 and lengthwise in an axial direction 35. The ridges 33 may be separated by a curved surface. The curved grooves 32 may have, but are not limited to having, a semi-circular curved surface. The plurality of axially extending grooves 32 may be aligned with each other, or one or more of the plurality of axially extending grooves 32 may be misaligned with each other. The grooves 32 may have the same depth radially inward from an outer surface 70 of the collar 30, or one or more, or all of the grooves 32 may have different depths into the collar 30. The collar 30 may be an elongated tube extending an entire length of the inner drive shaft 14 or for only a portion of the inner drive shaft 14. The elongated tube may be positioned concentrically about the inner drive shaft 14. The elongated tube may be formed from a material, such as, but not limited to, polytetrafluoroethylene (PTFE), which may be, but is not limited to being, TEFLON.

As shown in further examples of the disclosure not forming part of the claimed invention, in Figures 8 and 9, the instrument flushing system 16 may include one or more standoffs 28 extending between the inner surface 24 of the elongated outer housing 12 and the outer surface 26 of the inner drive shaft 14 to maintain the flushing fluid plenum 22. The standoff 28 need not contact both the outer surface 26 and the inner surface 24 at the same time. The standoff 28 of the instrument flushing system 16 may be formed from a collar 30 extending radially inward from the inner surface 24 of the elongated outer housing 12 whereby the collar 30 may include one or more axially extending grooves 32 therein. The collar 30 may include a plurality of axially extending grooves 32. The axially extending grooves 32 may be formed from a plurality of ridges 33 that extend inward in a radial direction 37 and lengthwise in an axial direction 35. The ridges 33 may be separated by a curved surface. The curved grooves 32 may have, but are not limited to having, a semi-circular curved surface. The plurality of axially extending grooves 32 may be aligned with each other, or one or more of the plurality of axially extending grooves 32 may be misaligned with each other. The grooves 32 may have the same depth radially inward from an outer surface 70 of the collar 30, or one or more, or all of the grooves 32 may have different depths into the collar 30. The collar 30 may be an elongated tube extending an entire length of the inner drive shaft 14 or for only a portion of the inner drive shaft 14. The elongated tube may be positioned concentrically about the inner drive shaft 14. The elongated tube may be formed from a material, such as, but not limited to, PTFE, which may be, but is not limited to being, TEFLON.

As shown in further examples of the disclosure not forming part of the claimed invention, in Figures 10 and 11, the instrument flushing system 16 may include one or more standoffs 28 extending between the inner surface 24 of the elongated outer housing 12 and the outer surface 26 of the inner drive shaft 14 to maintain the flushing fluid plenum 22. The standoff 28 need not contact both the outer surface 26 and the inner surface 24 at the same time. The standoff 28 of the instrument flushing system 16 may be formed from a collar 30 positioned in the hollow inner plenum 40. The collar 30 may include a plurality of ridges 33 extending inwardly and a plurality of ridges 33 extending outwardly. In particular, the collar 30 may include a plurality of axially extending grooves 32 are formed from a plurality of ridges 33 that extend outwardly in a radial direction 37 and a plurality of axially extending grooves 32 are formed from a plurality of ridges 33 that extend inwardly in a radial direction 37. The axially extending grooves 32 may be formed from a plurality of ridges 33 that extend inward in a radial direction 37 and lengthwise in an axial direction 35. The ridges 33 may be separated by a curved surface. The curved grooves 32 may have, but are not limited to having, a semi-circular curved surface. The plurality of axially extending grooves 32 may be aligned with each other, or one or more of the plurality of axially extending grooves 32 may be misaligned with each other. The grooves 32 may have the same depth radially inward from an outer surface 70 of the collar 30, or one or more, or all of the grooves 32 may have different depths into the collar 30. The collar 30 may be an elongated tube extending an entire length of the inner drive shaft 14 or for only a portion of the inner drive shaft 14. The elongated tube may be positioned concentrically about the inner drive shaft 14. The elongated tube may be formed from a material, such as, but not limited to, PTFE, which may be, but is not limited to being, TEFLON.

As shown in Figure in an example not forming part of the claimed invention, the instrument flushing system 16 may include inner drive shaft 14 positioned within the elongated outer housing 12 and extending into the hollow cavity 48 within the main body 38. The inner drive shaft 14 may be sized such that a flushing fluid plenum 22 exists in the hollow inner plenum 40 of the elongated outer housing 12 between an outer surface 26 of the inner drive shaft 14 and an inner surface 24 of the elongated outer housing 12 when the inner drive shaft 14 is positioned within the outer housing 12. The instrument flushing system 16 may include a standoff 28 formed from a collar 30. The collar 30 may include a smooth inner surface 110 and a smooth outer surface 112. The collar 30 may extend for all or a portion of a length of the inner drive shaft 14.

The instrument flushing system 16 may be configured to prevent or substantially limit leakage of a flushing fluid from the medical instrument 10 while a flushing fluid, such as, but not limited to, water, is passed through the instrument flushing system 16 within the medical instrument 10. The instrument flushing system 16 may include one or more seals 34, as shown in Figures 3, 4 and 6, to prevent leakage of the flushing fluid from the main body 38 where the first drive handle 60 extends from the main body 38. The seal 34 may be formed form any appropriate configuration, size and material or materials, such as, but not limited to polytetrafluoroethylene (PTFE), which may be, but is not limited to being, TEFLON. The seal 34, as shown in Figures 3, 4 and 6, may be formed from a first side 72 and a second side 74. The seal 34 may be formed from any appropriate manufacturing method. In at least one embodiment, the seal 34 may be formed from a stamping process in which the first and section sides 72, 74 may be stamped from a sheet of material. The first and second sides 72, 74 may include first and second sidewalls 76, 78 separated by a void sized to receive the first drive handle 60. When the first and second sides 72, 74 are positioned within the main body 38, the first and second sidewalls 76, 78 may contact each other to assist in sealing the hollow cavity 48 and to form a single opening 80 through which the first drive handle 60 extends. The seal 34 may be configured such that the first and second sides 72, 74 press against the first drive handle 60 tightly but not too tight that the friction prevents the first drive handle 60 from being moved relative to the main body 38. The first and second sides 72, 74 may have linear surfaces.

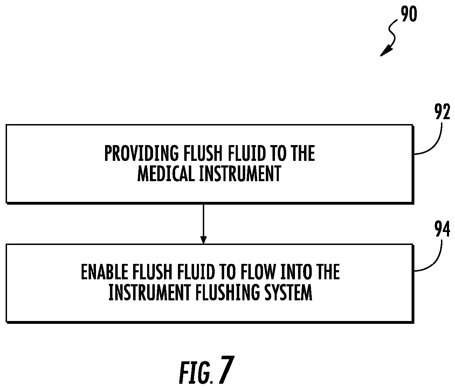

A method of cleaning a medical instrument at 90, as shown in Figure 7, may include cleaning a medical instrument 10, as previously set forth. In particular, the method 90 may include providing fluid to the medical instrument at 92. The medical instrument 10 may be any appropriate configuration, such as, but not limited to, an elongated outer housing 12 with a hollow inner plenum 40, whereby a distal end 42 of the elongated outer housing 12 includes an outer shaft distal opening 44. The medical instrument 10 may also include a main body 38 extending proximally from a proximal end 46 of the elongated outer housing 12. The medical instrument 10 may also include an inner drive shaft 14 positioned within the elongated outer housing 12 and extending into a hollow cavity 48 within the main body 38, whereby the inner drive shaft 14 is sized such that a flushing fluid plenum 22 exists in the hollow inner plenum 40 of the elongated outer housing 12 between an outer surface 26 of the inner drive shaft 14 and an inner surface 24 of the elongated outer housing 12. The medical instrument 10 may also include a drive system 36 in communication with the inner drive shaft 14 and extending radially outward from the main body 38, whereby the drive system 36 may be configured to move the inner drive shaft 14 axially within the hollow inner plenum 40 of the elongated outer housing 12. The medical instrument 10 may also include an instrument flushing system 16 in communication with the hollow cavity 48 within the main body 38 and configured to pass flushing fluid through the hollow cavity 48 within the main body 38 and through the flushing fluid plenum 22 in the hollow inner plenum 40 of the elongated outer housing 12 between the outer surface 26 of the inner drive shaft 14 and the inner surface 24 of the elongated outer housing 12.

The method 90 may also include enabling flush fluid to flow at 94 into the instrument flushing system 16. The step of enabling flush fluid to flow at 94 into the instrument flushing system 16 may include opening a valve (not shown), starting a pump (not shown) in fluid communication with the instrument flushing system 16, depressing a plunger within a syringe 25 in fluid communication with the instrument flushing system 16 and the like. Once flushing fluid is delivered to the instrument flushing system 16, such as into at least one port 18 of the instrument flushing system 16 at a proximal end 20 of the main body 38 of the medical instrument 10, the fluid fills the hollow cavity 48 within the main body 38 of the medical instrument 10. The flushing fluid flows from the hollow cavity 48 within the main body 38 of the medical instrument 10 and into the flushing fluid plenum 22 within the elongated outer housing 12. When flowing through the flushing fluid plenum 22, the flushing fluid will pass through one or more axially extending grooves 32 in a standoff 28, such as, but not limited to, the collar 30. The standoff 28, such as, but not limited to, the collar 30 positions the inner drive shaft 14 within the elongated outer housing 12 to maintain the flushing fluid plenum 22 to prevent inner drive shaft 14 from blocking the flushing fluid plenum 22. The flushing fluid may be exhausted from the flushing fluid plenum 22 and carry with it tissue and blood found within the flushing fluid plenum 22 and at the one more working heads 52.

The step of providing fluid to the medical instrument 10 at 92 may include providing fluid to the medical instrument 10 via at least one port 18 of the instrument flushing system 16 at a proximal end 20 of the main body 38 of the medical instrument 10. The step of providing fluid to the medical instrument 10 at 92 may also include attaching the medical instrument 10 to a fluid source, which may be, but is not limited to being, a syringe, a tube connected to a pressurized fluid source and the like. The step of providing fluid to the medical instrument 10 at 92 may include providing fluid to the medical instrument 10 via a releasable connection at least one port 18. The step of providing fluid to the medical instrument 10 at 92 may include providing fluid to the medical instrument 10 including at least one standoff 28 positioned in the flushing fluid plenum 22 and extending between the outer surface 26 of the inner drive shaft 14 and the inner surface 24 of the elongated outer housing 12 to maintain the flushing fluid plenum 22. The step of providing fluid to the medical instrument 10 at 92 may also include providing fluid to the medical instrument 10 wherein the standoff 28 of the instrument flushing system 16 is formed from a collar 30 extending radially outward from the outer surface 26 of the inner drive shaft 14, whereby the collar 30 includes one or more axially extending grooves 32 therein. The step of providing fluid to the medical instrument 10 at 92 may include providing fluid to the medical instrument 10 wherein the standoff 28 is formed from a collar 30 extending radially outward from the outer surface 26 of the inner drive shaft 14, wherein the collar 30 includes a plurality of axially extending grooves 32 positioned in a radially outer surface 70 of the collar 30. The step of providing fluid to the medical instrument 10 at 92 may include providing fluid to the medical instrument 10 wherein the collar 30 includes a plurality of axially extending grooves 32 that are aligned with each other. The step of providing fluid to the medical instrument 10 at 92 may include providing fluid to the medical instrument 10 wherein the drive system 36 is formed from a first drive handle 60 and a second drive handle 62 configured to be movable relative to each other to move the inner drive shaft 14 axially and the instrument flushing system 16 further includes at least one seal 34 configured to seal the hollow cavity 48 within the main body 38 where the first drive handle 60 extends radially outward from the main body 38.

## Claims

1. A medical instrument (10), comprising:
an elongated outer housing (12) with a hollow inner plenum (40), wherein a distal end (42) of the elongated outer housing (12) includes an outer shaft distal opening (44);
a main body (38) extending proximally from a proximal end (46) of the elongated outer housing (12);
an inner drive shaft (14) positioned within the elongated outer housing (12) and extending into a hollow cavity (48) within the main body (38), wherein the inner drive shaft (14) is sized such that a flushing fluid plenum (40) exists in the hollow inner plenum (40) of the elongated outer housing (12) between an outer surface (26) of the inner drive shaft (14) and an inner surface (24) of the elongated outer housing (12);
a drive system (36) in communication with the inner drive shaft (14) and extending radially outward from the main body (38), wherein the drive system (36) is configured to move the inner drive shaft (14) axially within the hollow inner plenum (40) of the elongated outer housing (12); and
an instrument flushing system (16) in communication with the hollow cavity (48) within the main body (38) and configured to pass flushing fluid through the hollow cavity (48) within the main body (38) and through the flushing fluid plenum (40) in the hollow inner plenum (40) of the elongated outer housing (12) between the outer surface (26) of the inner drive shaft (14) and the inner surface (24) of the elongated outer housing (12);
**characterized in that** the instrument flushing system (16) further comprises at least one standoff (28) positioned in the flushing fluid plenum (40) and extending between the outer surface (26) of the inner drive shaft (14) and the inner surface (24) of the elongated outer housing (12) to maintain the flushing fluid plenum (40);
wherein the at least one standoff (28) of the instrument flushing system (16) is formed from a collar (30) extending radially outward from the outer surface (26) of the inner drive shaft (14), wherein the collar (30) includes at least one axially extending groove (32) therein positioned on a radially outer surface of the collar (30).

2. The medical instrument (10) of claim 1, wherein the instrument flushing system (16) further comprises at least one port (18) at a proximal end (20) of the main body (38) of the medical instrument (10).

3. The medical instrument (10) of claim 2, wherein the at least one port (18) of the instrument flushing system (16) is a releasable connection.

4. The medical instrument (10) of claim 1, wherein the at least one axially extending groove (32) is one of a plurality of axially extending grooves (32).

5. The medical instrument (10) of claim 4, wherein the plurality of axially extending grooves (32) are aligned with each other and are positioned in a radially outer surface of the collar (30).

6. The medical instrument (10) of claim 1, wherein the collar (30) is an elongated tube positioned concentrically about the inner drive shaft (14).

7. The medical instrument (10) of claim 1, wherein the drive system (36) is formed from a first drive handle (60) and a second drive handle (62) configured to be movable relative to each other to move the inner drive shaft (14) axially.

8. The medical instrument (10) of claim 7, wherein the instrument flushing system (16) further comprises at least one seal (34) configured to seal the hollow cavity (48) within the main body (38) where the first and second drive handles (60, 62) extend radially outward from the main body (38).

9. A method (90) of cleaning a medical instrument (10), comprising:
providing fluid to the medical instrument (10), comprising:
an elongated outer housing (12) with a hollow inner plenum (40), wherein a distal end (42) of the elongated outer housing (12) includes an outer shaft distal opening (44);
a main body (38) extending proximally from a proximal end (46) of the elongated outer housing (12);
an inner drive shaft (14) positioned within the elongated outer housing (12) and extending into a hollow cavity (48) within the main body (38), wherein the inner drive shaft (14) is sized such that a flushing fluid plenum (40) exists in the hollow inner plenum (40) of the elongated outer housing (12) between an outer surface (26) of the inner drive shaft (14) and an inner surface (24) of the elongated outer housing (12);
a drive system (36) in communication with the inner drive shaft (14) and extending radially outward from the main body (38), wherein the drive system (36) is configured to move the inner drive shaft (14) axially within the hollow inner plenum (40) of the elongated outer housing (12); and
an instrument flushing system (16) in communication with the hollow cavity (48) within the main body (38) and configured to pass flushing fluid through the hollow cavity (48) within the main body (38) and through the flushing fluid plenum (40) in the hollow inner plenum (40) of the elongated outer housing (12) between the outer surface (26) of the inner drive shaft (14) and the inner surface (24) of the elongated outer housing (12);
**characterized in that** the instrument flushing system (16) further comprises at least one standoff (28) positioned in the flushing fluid plenum (40) and extending between the outer surface (26) of the inner drive shaft (14) and the inner surface (24) of the elongated outer housing (12) to maintain the flushing fluid plenum (40);
wherein the at least one standoff (28) of the instrument flushing system (16) is formed from a collar (30) extending radially outward from the outer surface (26) of the inner drive shaft (14), wherein the collar (30) includes at least one axially extending groove (32) therein positioned on a radially outer surface of the collar (30); and
enabling flush fluid to flow into the instrument flushing system (16).

10. The method (90) of cleaning a medical instrument (10) of claim 9, wherein providing fluid to the medical instrument (10) comprises providing fluid to the medical instrument (10) via at least one port (18) of the instrument flushing system (16) at a proximal end (20) of the main body (38) of the medical instrument (10).

11. The method (90) of cleaning a medical instrument (10) of claim 10, wherein providing fluid to the medical instrument (10) via the at least one port (18) of the instrument flushing system (16) comprises providing fluid to the medical instrument (10) via a releasable connection at the at least one port (18) of the instrument flushing system (16) at a proximal end (20) of the main body (38) of the medical instrument (10).

12. The method (90) of cleaning a medical instrument (10) of claim 9, wherein providing fluid to the medical instrument (10) comprises providing fluid to the medical instrument (10) wherein the drive system (36) is formed from a first drive handle (60) and a second drive handle (62) configured to be movable relative to each other to move the inner drive shaft (14) axially and the instrument flushing system (16) further comprises at least one seal (34) configured to seal the hollow cavity (48) within the main body (38) where the first and second drive handles (60, 62) extend radially outward from the main body (38).

## Patentansprüche

1. Medizinisches Instrument (10), das aufweist:
ein längliches Außengehäuse (12) mit einem hohlen inneren Zwischenraum (40), wobei ein distales Ende (42) des länglichen Außengehäuses (12) eine distale Außenschaftöffnung (44) enthält;
einen Hauptkörper (38), der sich proximal von einem proximalen Ende (46) des länglichen Außengehäuses (12) erstreckt;
eine innere Antriebswelle (14), die innerhalb des länglichen Außengehäuses (12) positioniert ist und sich in eine hohle Kavität (48) innerhalb des Hauptkörpers (38) erstreckt, wobei die innere Antriebswelle (14) so bemessen ist, dass in dem hohlen inneren Zwischenraum (40) des länglichen Außengehäuses (12) zwischen einer äußeren Oberfläche (26) der inneren Antriebswelle (14) und einer inneren Oberfläche (24) des länglichen Außengehäuses (12) ein Spülfluid-Zwischenraum (40) existiert;
ein Antriebssystem (36), das mit der inneren Antriebswelle (14) kommuniziert und sich von dem Hauptgehäuse (38) radial nach außen erstreckt, wobei das Antriebssystem (36) dazu ausgebildet ist, die innere Antriebswelle (14) axial innerhalb des hohlen inneren Zwischenraums (40) des länglichen Außengehäuses (12) zu bewegen, und
ein Instrumentenspülsystem (16), das mit der hohlen Kavität (48) innerhalb des Hauptkörpers (38) kommuniziert und dazu ausgebildet ist, Spülfluid durch die hohle Kavität (48) innerhalb des Hauptkörpers (38) und durch den Spülfluidzwischenraum (40) in dem hohlen inneren Zwischenraum (40) des länglichen Außengehäuses (12) zwischen der äußeren Oberfläche (26) der inneren Antriebswelle (14) und der inneren Oberfläche (24) des länglichen Außengehäuses (12) zu leiten;
**dadurch gekennzeichnet, dass** das Instrumentenspülsystem (16) weiterhin zumindest einen Abstandhalter (28), der in dem Spülfluidzwischenraum (40) positioniert ist und sich zwischen der äußeren Oberfläche (26) der inneren Antriebswelle (14) und der inneren Oberfläche (24) des länglichen Außengehäuses (12) erstreckt, um den Spülfluidzwischenraum (40) aufrechtzuerhalten, aufweist,
wobei der zumindest eine Abstandhalter (28) des Instrumentenspülsystems (16) aus einem Kragen (30), der sich von der äußeren Oberfläche (26) der inneren Antriebswelle (14) radial nach außen erstreckt, gebildet ist, wobei der Kragen (30) zumindest eine sich axial erstreckende Nut (32), die auf einer radial äußeren Oberfläche des Kragens (30) angeordnet ist, darin enthält.

2. Medizinisches Instrument (10) nach Anspruch 1, wobei das Instrumentenspülsystem (16) weiterhin zumindest einen Port (18) an einem proximalen Ende (20) des Hauptkörpers (38) des medizinischen Instruments (10) aufweist.

3. Medizinisches Instrument (10) nach Anspruch 2, wobei der zumindest eine Port (18) des Instrumentenspülsystems (16) eine lösbare Verbindung ist.

4. Medizinisches Instrument (10) nach Anspruch 1, wobei die zumindest eine sich axial erstreckende Nut (32) eine von mehreren sich axial erstreckenden Nuten (32) ist.

5. Medizinisches Instrument (10) nach Anspruch 4, wobei die mehreren sich axial erstreckenden Nuten (32) miteinander ausgerichtet sind und in einer radial äußeren Oberfläche des Kragens (30) angeordnet sind.

6. Medizinisches Instrument (10) nach Anspruch 1, wobei der Kragen (30) eine längliche Röhre ist, die konzentrisch um die innere Antriebswelle (14) positioniert ist.

7. Medizinisches Instrument (10) nach Anspruch 1, wobei das Antriebssystem (36) aus einem ersten Ansteuerungsgriff (60) und einem zweiten Ansteuerungsgriff (62), die dazu ausgebildet sind, relativ zueinander beweglich zu sein, um die innere Antriebswelle (14) axial zu bewegen, gebildet ist.

8. Medizinisches Instrument (10) nach Anspruch 7, wobei das Instrumentenspülsystem (16) weiterhin zumindest eine Dichtung (34) aufweist, die dazu ausgebildet ist, die hohle Kavität (48) innerhalb des Hauptkörpers (38), wo sich der erste und der zweite Ansteuerungsgriff (60, 62) von dem Hauptkörper (38) radial nach außen erstrecken, abzudichten.

9. Verfahren (90) zum Reinigen eines medizinischen Instruments (10), das aufweist:
Zuführen von Fluid zu dem medizinischen Instrument (10), das aufweist:
ein längliches Außengehäuse (12) mit einem hohlen inneren Zwischenraum (40), wobei ein distales Ende (42) des länglichen Außengehäuses (12) eine distale Außenschaftöffnung (44) enthält;
einen Hauptkörper (38), der sich proximal von einem proximalen Ende (46) des länglichen Außengehäuses (12) erstreckt;
eine innere Antriebswelle (14), die innerhalb des länglichen Außengehäuses (12) positioniert ist und sich in eine hohle Kavität (48) innerhalb des Hauptkörpers (38) erstreckt, wobei die innere Antriebswelle (14) so bemessen ist, dass in dem hohlen inneren Zwischenraum (40) des länglichen Außengehäuses (12) zwischen einer äußeren Oberfläche (26) der inneren Antriebswelle (14) und einer inneren Oberfläche (24) des länglichen Außengehäuses (12) ein Spülfluid-Zwischenraum (40) existiert;
ein Antriebssystem (36), das mit der inneren Antriebswelle (14) kommuniziert und sich von dem Hauptgehäuse (38) radial nach außen erstreckt, wobei das Antriebssystem (36) dazu ausgebildet ist, die innere Antriebswelle (14) axial innerhalb des hohlen inneren Zwischenraums (40) des länglichen Außengehäuses (12) zu bewegen, und
ein Instrumentenspülsystem (16), das mit der hohlen Kavität (48) innerhalb des Hauptkörpers (38) kommuniziert und dazu ausgebildet ist, Spülfluid durch die hohle Kavität (48) innerhalb des Hauptkörpers (38) und durch den Spülfluidzwischenraum (40) in dem hohlen inneren Zwischenraum (40) des länglichen Außengehäuses (12) zwischen der äußeren Oberfläche (26) der inneren Antriebswelle (14) und der inneren Oberfläche (24) des länglichen Außengehäuses (12) zu leiten;
**dadurch gekennzeichnet, dass** das Instrumentenspülsystem (16) weiterhin zumindest einen Abstandhalter (28), der in dem Spülfluidzwischenraum (40) positioniert ist und sich zwischen der äußeren Oberfläche (26) der inneren Antriebswelle (14) und der inneren Oberfläche (24) des länglichen Außengehäuses (12) erstreckt, um den Spülfluidzwischenraum (40) aufrechtzuerhalten, aufweist,
wobei der zumindest eine Abstandhalter (28) des Instrumentenspülsystems (16) aus einem Kragen (30), der sich von der äußeren Oberfläche (26) der inneren Antriebswelle (14) radial nach außen erstreckt, gebildet ist, wobei der Kragen (30) zumindest eine sich axial erstreckende Nut (32), die auf einer radial äußeren Oberfläche des Kragens (30) angeordnet ist, darin enthält.
Ermöglichen, dass Spülfluid ermöglicht, in das Instrumentenspülsystem (16) fließt.

10. Verfahren (90) zum Reinigen eines medizinischen Instruments (10) nach Anspruch 9, wobei das Zuführen von Fluid zu dem medizinischen Instrument (10) das Zuführen von Fluid zu dem medizinischen Instrument (10) über zumindest einen Port (18) des Instrumentenspülsystems (16) an einem proximalen Ende (20) des Hauptkörpers (38) des medizinischen Instruments (10) aufweist.

11. Verfahren (90) zum Reinigen eines medizinischen Instruments (10) nach Anspruch 10, wobei das Zuführen von Fluid zu dem medizinischen Instrument (10) über den zumindest einen Port (18) des Instrumentenspülsystems (16) das Zuführen von Fluid zu dem medizinischen Instrument (10) über eine lösbare Verbindung an dem zumindest einen Port (18) des Instrumentenspülsystems (16) an einem proximalen Ende (20) des Hauptkörpers (38) des medizinischen Instruments (10) aufweist.

12. Verfahren (90) zum Reinigen eines medizinischen Instruments (10) nach Anspruch 9, wobei das Zuführen von Fluid zu dem medizinischen Instrument (10) das Bereitstellen von Fluid zu dem medizinischen Instrument (10) aufweist, wobei das Antriebssystem (36) aus einem ersten Ansteuerungsgriff (60) und einem zweiten Ansteuerungsgriff (62) gebildet ist, die dazu ausgebildet sind, relativ zueinander beweglich zu sein, um die innere Antriebswelle (14) axial zu bewegen, und wobei das Instrumentenspülsystem (16) weiterhin zumindest eine Dichtung (34) aufweist, die dazu ausgebildet ist, die hohle Kavität (48) innerhalb des Hauptkörpers (38), wo sich der erste und der zweite Ansteuerungsgriff (60, 62) von dem Hauptkörper (38) radial nach außen erstrecken, abzudichten.

## Revendications

1. Instrument médical (10) comprenant :
- un boîtier extérieur allongé (12) avec un plénum intérieur (40), creux, le boîtier extérieur allongé (12) ayant une extrémité distale (42) avec une ouverture distale de boîtier extérieur (44),
- un corps principal (38) issu sensiblement d'une extrémité proximale (46) du boîtier extérieur allongé (12),
- une tige intérieure d'entraînement (14) logée dans le boîtier extérieur allongé (12) et passant dans la cavité (48) du corps principal (38), la tige d'entraînement intérieur (14) étant dimensionnée de façon à laisser un plénum de fluide de rinçage (40) dans le plénum intérieur creux (40) du boîtier extérieur allongé (12) entre la surface extérieure (26) de la tige intérieure d'entraînement (14) et la surface intérieure (24) du boîtier extérieur allongé (12),
- un système d'entraînement (36) en liaison avec la tige intérieure d'entraînement (14) et venant radialement vers l'extérieur du corps principal (38), le système d'entraînement (36) étant configuré pour déplacer axialement la tige intérieure d'entraînement (14) dans le plénum intérieur creux (40) du boîtier extérieur allongé (12), et
- un système de rinçage d'instrument (16) en communication avec la cavité (48) du corps principal (38) et configuré pour faire passer le fluide de rinçage à travers la cavité (48) du corps principal (38) et à travers le plénum de fluide de rinçage (40) du plénum intérieur creux (40) du boîtier extérieur allongé (12) entre la surface extérieure (26) de la tige intérieure d'entraînement (14) et la surface intérieure (24) du boîtier extérieur allongé (12),
instrument **caractérisé en ce que**
le système de rinçage d'instrument (16) comprend en outre au moins une butée (28) dans le plénum de fluide de rinçage (40) et s'étendant entre la surface extérieure (26) de la tige intérieure d'entraînement (14) et la surface intérieure (24) du boîtier extérieur allongé (12) pour maintenir le plénum de fluide de rinçage (40),
dans lequel
au moins la butée (28) du système de rinçage d'instrument (16) est formée à partir du col (30) s'étendant radialement vers l'extérieur à partir de la surface extérieure (26) de la tige intérieure d'entraînement (14),
- le col (30) comprenant au moins une rainure (32) s'étendant axialement et située sur la surface radiale extérieure du col (30).

2. Instrument médical (10) selon la revendication 1,
dans lequel
le système de rinçage d'instrument (16) comprend en outre au moins un port (18) à l'extrémité proximale (20) du corps principal (38) de l'instrument médical (10).

3. Instrument médical (10) selon la revendication 2,
dans lequel
le port (18) du système de rinçage d'instrument (16) est une connexion détachable.

4. Instrument médical (10) selon la revendication 1,
dans lequel
au moins la rainure (32) s'étendant axialement est l'une d'un ensemble de rainures (32) s'étendant axialement.

5. Instrument médical (10) selon la revendication 4,
dans lequel
les rainures de l'ensemble des rainures (32) s'étendant axialement sont alignées l'une sur l'autre et sont dans une surface radialement extérieure du col (30).

6. Instrument médical (10) selon la revendication 1,
dans lequel
le col (30) est un tube allongé entourant de manière concentrique la tige intérieure d'entraînement (14).

7. Instrument médical (10) selon la revendication 1,
dans lequel
le système d'entraînement (36) est formé d'une première poignée d'actionnement (60) et d'une seconde poignée d'actionnement (62) mobiles l'une par rapport à l'autre pour déplacer axialement la tige intérieure d'entraînement (14).

8. Instrument médical (10) selon la revendication 7,
dans lequel
le système de rinçage d'instrument (16) comprend en outre au moins un joint (34) pour réaliser l'étanchéité de la cavité creuse (48) dans le corps principal (38), la première et la seconde poignées d'actionnement (60, 62) s'étendant radialement vers l'extérieur à partir du corps principal (38).

9. Procédé (90) de nettoyage d'un instrument médical (10) consistant à fournir un fluide à l'instrument médical (10) comprenant :
- un boîtier extérieur allongé (12) avec un plénum intérieur creux (40), l'extrémité distale (42) du boîtier extérieur allongé (12) ayant une ouverture distale de boîtier extérieur (44),
- un corps principal (38) issu sensiblement de l'extrémité proximale (46) du boîtier extérieur allongé (12),
- une tige intérieure d'entraînement (14) logée dans le boîtier extérieur allongé (12) et passant dans la cavité creuse (48) du corps principal (38), la tige intérieure d'entraînement (14) étant dimensionnée de façon à laisser un plénum de fluide de rinçage (40) dans le plénum intérieur creux (40) du boîtier extérieur allongé (12) entre la surface extérieure (26) de la tige intérieure d'entraînement (14) et la surface intérieure (24) du boîtier extérieur allongé (12),
- un système d'entraînement (36) en liaison avec la tige intérieure d'entraînement (14) et s'étendant radialement vers l'extérieur à partir du corps principal (38), le système d'entraînement (36) étant configuré pour déplacer axialement la tige intérieure d'entraînement (14) dans le plénum intérieur creux (40) du boîtier extérieur allongé (12), et
- un système de rinçage d'instrument (16) en liaison avec la cavité (48) du corps principal (38) et configuré pour faire passer le fluide de rinçage à travers la cavité (48) dans le corps principal (38) et à travers le plénum de fluide de rinçage (40) dans le plénum intérieur creux (40) du boîtier extérieur allongé (12) entre la surface extérieure (26) de la tige intérieure d'entraînement (14) et la surface intérieure (24) du boîtier extérieur allongé (12),
procédé **caractérisé en ce que**
le système de rinçage d'instrument (16) comprend en outre au moins une butée (28) dans le plénum de fluide de rinçage (40) et s'étendant entre la surface extérieure (26) de la tige intérieure d'entraînement (14) et la surface intérieure (24) du boîtier extérieur allongé (12) pour maintenir le plénum de fluide de rinçage (40),
dans lequel
au moins une butée (28) du système de rinçage d'instrument (16) est formée à partir d'un col (30) s'étendant radialement vers l'extérieur à partir de la surface extérieure (26) de la tige intérieure d'entraînement (14), le col (30) ayant au moins une rainure axiale (32) sur la surface radiale extérieure du col (30), et
permettant au liquide de rinçage de passer sur le système de rinçage d'instrument (16).

10. Procédé (90) de nettoyage d'un instrument médical (10) selon la revendication 9,
dans lequel
alimenter en fluide l'instrument médical (10) consiste à fournir le fluide à l'instrument médical (10) par au moins un port (18) du système de rinçage d'instrument (16) à l'extrémité proximale (20) du corps principal (38) de l'instrument médical (10).

11. Procédé (90) de nettoyage d'un instrument médical (10) selon la revendication 10,
selon lequel
alimenter en fluide l'instrument médical (10) par au moins un port (18) du système de rinçage d'instrument (16) consiste à alimenter en fluide l'instrument médical (10) par une liaison détachable à un port (18) du système de rinçage d'instrument (16) à l'extrémité proximale (20) du corps principal (38) de l'instrument médical (10).

12. Procédé (90) de nettoyage d'un instrument médical (10) selon la revendication 9,
dans lequel
alimenter en fluide l'instrument médical (10) consiste à fournir du fluide à l'instrument médical (10), dont
le système d'entraînement (36) est formé d'au moins une première poignée d'actionnement (60) et d'une seconde poignée d'actionnement (62) mobiles l'une par rapport à l'autre et déplaçant la tige d'entraînement intérieur (14) axialement, et le système de rinçage d'instrument (16) comprend en outre au moins un joint (34) pour réaliser l'étanchéité de la cavité creuse (48) dans le corps principal (38), la première et la seconde poignée d'actionnement (60, 62) venant radialement vers l'extérieur à partir du corps principal (38).
